# EUROPEAN PATENT SPECIFICATION

(11) **EP 3 761 916 B1**
(45) Date of publication and mention of the grant of the patent: **01.05.2024**
(21) Application number: 19764868.6
(22) Date of filing: 06.03.2019
(51) Int. Cl.: A61F 2/50, A61F 2/54, A61F 2/58, A61F 2/68, A61F 2/70, A61F 2/72, A61B 5/389, A61B 5/11

(54) **GRASP ASSISTANCE SYSTEM AND METHOD**
GREIFHILFESYSTEM UND -VERFAHREN
SYSTÈME ET PROCÉDÉ D'AIDE À LA PRÉHENSION

(30) Priority: 09.03.2018 US 201862640609 P
(43) Date of publication of application: 13.01.2021
(73) Proprietor: Myomo, Inc., Cambridge, MA 02142 (US)
(72) Inventor: KESNER, Samuel, Arlington, Massachusetts 02476 (US); PEISNER, Jeffrey, Durham, NC 27713 (US); TACY, Gene, Windham, New Hampshire 03087 (US); HARLAN, Andrew, Somerville, Massachusetts 02143 (US)
(74) Representative: Lucke, Andreas
(86) International application number: PCT/US2019/020874
(87) International publication number: WO 2019/173422

(56) References cited:
- WO-A1-2015/047070
- WO-A1-2016/205356
- US-A1- 2008 071 386
- US-A1- 2008 215 162
- US-A1- 2013 253 705
- US-A1- 2014 200 432
- US-A1- 2015 141 773

## Description

This application claims priority from U.S. Provisional Patent Application 62/640,609, filed March 9, 2018.

### TECHNICAL FIELD

The present invention relates to control of powered orthotic devices, and more specifically, to controlling such devices to assist a user with performing grasping movement tasks.

### BACKGROUND ART

Survivors of stroke, brain injury, and other neuromuscular trauma or disease (e.g., Amyotrophic Lateral Sclerosis (ALS), Multiple Sclerosis (MS), Muscular Dystrophy (MD), etc.) are often left with hemipareisis or severe weakness in some parts of the body. The result can be impaired or lost function in one or more limbs. But people can rehabilitate significantly from many of the impairments following such neurological traumas, and such rehabilitation can be more effective and motor patterns can be re-learned more quickly if a rehabilitative exercise regime includes the execution of familiar functional tasks. Following neuromuscular trauma, however, the control or strength in an afflicted limb or limbs may be so severely diminished that the patient may have difficulty (or be unable) performing constructive functional rehabilitation exercises without assistance.

U.S. Patents 8585620, 8926534 and 9398994 describe examples of powered orthotic devices to assist those with neuromuscular problems. US 2013/0253705 A1 discloses a method of operating a grasping device using a plurality of bi-directional state flow maps, wherein each state flow map defines a sequence of poses for a plurality of joints in the grasping device and a user-generated control signal is used to select one of the state flow maps and to traverse the respective sequence of poses. But even given such advanced solutions, control of these devices for common movement tasks such as hand grasping functionality remains a challenge.

### SUMMARY

The present invention provides a computer-implemented method for controlling a grasp control system according to claim 1 and a computer-implemented grasp control system according to claim 8. Examples thereof are detailed in the dependent claims. Embodiments of the present invention are directed to a computer-implemented method that employs at least one hardware implemented computer processor for controlling a grasp control system to assist an operator with a grasping movement task. The at least one hardware processor is operated to execute program instructions for: monitoring a movement intention signal of a grasping movement muscle of the operator, identifying a volitional operator input for the grasping movement task from the movement intention signal, and operating a powered orthotic device based on the volitional operator input to perform the grasping movement task as a chain of motion primitives, wherein each motion primitive is a fundamental unit of grasping motion defined along a movement path with a single degree of freedom. The motion primitives are configured to be combined in parallel to form the chain of motion primitives.

In specific embodiments, operating the powered orthotic device includes performing the grasping movement task as chain of motion primitives at a variable speed controlled as a function of the volitional operator input. A second movement intention signal of a second grasping movement muscle of the wearer may be monitored, and the volitional operator input then may be identified from both movement intention signals. For example, the grasping movement muscles monitored by the movement intention signals may be antagonistic muscles.

Performing the grasping movement task may include undoing a portion of the grasping movement task based on the volitional operator input by performing a portion of the chain of motion primitives in reverse order. A finger force signal may be generated by one or more fingers of the wearer related to the grasping movement task, and then monitored so that the volitional operator input is identified from the movement intention signal and the finger force signal.

The chain of motion primitives may create grasping motion with at least two degrees of freedom. The chain of motion primitives may be predefined system chains and/or user defined chains, e.g., dynamically defined by the user. The movement intention signal may be an electromyography (EMG) signal, or muscle twitch, pressure, force, etc.

Embodiments of the present invention also include a computer-implemented grasp control system for assisting an operator with a grasping movement task. The system includes
a muscle movement sensor that is configured for monitoring a grasping movement muscle of the operator to produce a movement intention signal. A powered orthotic device is configured for assisting grasping motion of the operator. Data storage memory is configured for storing grasp control software, the movement intention signal, and other system information. A grasp control processor including at least one hardware processor is coupled to the data storage memory and configured to execute the grasp control software. The grasp control software includes processor readable instructions to implement a grasp control algorithm for: identifying a volitional operator input for the grasping movement task from the movement intention signal, and operation of the powered orthotic device is based on the volitional operator input to perform the grasping movement task as a chain of motion primitives, wherein each motion primitive is a fundamental unit of grasping motion defined along a movement path with a single degree of freedom. The motion primitives are configured to be combined in parallel to form the chain of motion primitives.

The grasp control algorithm may operate the powered orthotic device to perform the grasping movement task as chain of motion primitives at a variable speed controlled as a function of the volitional operator input. There may also be a second muscle movement sensor that is configured for monitoring a second grasping movement muscle of the operator to produce a second movement intention signal, wherein the grasp control algorithm identifies the volitional operator input from both movement intention signals. For example, the grasping movement muscles may be antagonistic muscles.

Performing the grasping movement task may include undoing a portion of the grasping movement task based on the volitional operator input by performing a portion of the chain of motion primitives in reverse order. There may also be a finger force sensor that is configured for monitoring a finger force signal generated by one or more fingers of the wearer related to the grasping movement task, wherein the grasp control algorithm identifies the volitional operator input from the movement intention signal and the finger force signal.

The chain of motion primitives may create grasping motion with at least two degrees of freedom. The chain of motion primitives may be predefined system chains and/or user defined chains, e.g., dynamically defined by the user. The muscle movement sensor may be an electromyography (EMG) signal sensor.

### BRIEF DESCRIPTION OF THE DRAWINGS

Figure 1 shows various functional blocks in a grasp control system for a powered orthotic device according to an embodiment of the present invention.
Figure 2 shows various functional block details of a user interface for a grasp control system according to an embodiment of the present invention.
Figures 3A-3G show example photographs of a user fitted with a powered orthotic device that he uses for the specific grasping movement task of lifting a cup for drinking.
Figure 4 shows a graph of various relevant parameters during the process shown in Figs. 3A-3G.
Figure 5 shows an example of how a motion chain is shaped in the case of single direction scrubbing.
Figure 6 shows a similar set of waveforms for another example with a single DOF scrubbed at a varying speed using two inputs, a positive and negative direction VOI.
Figure 7 shows an example of the structure of a powered orthotic device suitable for assisting a user with performing a hand task movement according to an embodiment of the present invention.
Figure 8 is grasp-release flowchart showing various logical steps in a grasp control process using motion chains according to an embodiment of the present invention.
Figure 9 shows various example waveforms associated with a basic precontact-secure-hold sequence.
Figure 10 shows various example waveforms associated with a basic precontact-secure-hold/ratcheting sequence.
Figure 11 shows various example waveforms associated with a single sensor precontact-trigger release-release process.
Figure 12 shows various example waveforms associated with a two sensor precontact-trigger release-release process.
Figure 13 shows various example waveforms for grasp slipping for a single flexor muscle sensor.
Figure 14 shows various example waveforms for grasp releasing for a single flexor muscle sensor.
Figure 15 shows various example waveforms for grasp releasing for dual flexor sensors.
Figure 16 illustrates operation that provides enhanced functionality for other external devices in order to complete an action the other device is unable to achieve by itself.
Figure 17 shows one specific logical flow arrangement for acquiring new task motion chains.

### DETAILED DESCRIPTION

Various embodiments of the present invention are directed to techniques for grasping control to perform grasp movement tasks with a powered orthotic device.

### Definitions:

"Scrubbing" refers to traversing forward or backward through a command set or signal.

"Volitional Operator Input (VOI)" refers to a system control input that is controlled by user intent; for example, an electromyography (EMG) signal input, an electroencephalogram (EEG) signal input, or a body-worn linear transducer input.

"Degree of Freedom (DOF)" is an independent direction in which motion can occur about a translational or rotational joint or combination thereof. For example, a human wrist contains 3 DOF while an elbow only contains 1.

A "motion primitive" is a fundamental unit of motion involving a single DOF moving along a linear or non-linear movement path through a prescribed position, velocity, or force target trajectory.

A "motion chain" is a set of motion primitives that are connected in parallel to create a more complex action across one or more DOF.

A "shaped motion chain (SMC)" is a motion chain that is traversed at variable speed based on VOI input.

Complex motions that are too sophisticated for an average user to execute in real time can be efficiently created and played back by chaining together multiple simple movements so as to form a more complex series of movements. This also allows for scenarios where the number of device sensors is fewer than the number of DOF's. For example, a therapist can come into a user's home and help them record complex tasks like opening their specific kitchen drawer or reaching for the handle for their model of refrigerator. The user can then later activate these custom routines during daily activities, allowing them more independence at home in daily life. Chaining complicated motions together for more complex therapeutic tasks such as coordinated arm-hand lifts and pick-and-place tasks also could be beneficial for more impaired users in therapy. The following discussion is presented in terms of "grasping" tasks and functions, but the present invention is not limited to that specific application, and the approach of chain together sequences of simpler motions can usefully be applied to other movement tasks with multiple DOFs.

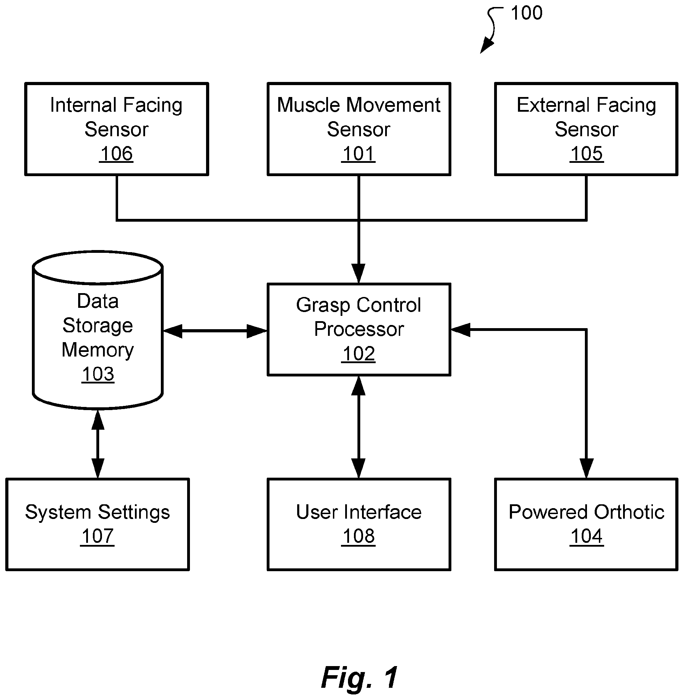

Embodiments of the present invention, as shown in Figure 1, are directed to a computer-implemented grasp control system **100** and related methods for controlling a powered orthotic **104** to assist an operator with a grasping movement task as a chain of motion primitives, for example, as a shaped motion chain SMC. The grasp control system **100** estimates that state of the user and the powered orthotic **104** and, based on system operation mode, user history, shared usage information and other data, determines the intended next motion in the chain of motion primitives and outputs corresponding control commands to the powered orthotic **104** device. Chains of motion primitives may perform more complicated grasping motions including those with at least two degrees of freedom. The chain of motion primitives may specifically be predefined system chains and/or user defined chains, e.g., dynamically defined by the user.

A muscle movement sensor **101,** e.g., an electromyography (EMG) signal sensor, EEG sensor, muscle contraction sensors, etc., is configured for monitoring a grasping movement muscle of the operator to produce a movement intention signal that represents a Volitional Operator Input (VOI) to the system. Besides an EMG sensor, the muscle movement sensor **10** that produces a given VOI may include without limitation an EEG sensor, a linear transducer input, a suck-and-puff tube, or other physiological user-controlled input.

There also may be one or more other additional data sensors **106** configured to produce useful information signals such as EMG?, IMU, position, joint angles, force, strain, etc. For example, the additional data sensor **106** may include a second muscle movement sensor that is configured for monitoring a second grasping movement muscle of the operator to produce a second movement intention signal (for example, the grasping movement muscles may be antagonistic muscles). Or the additional data sensor **106** may be a finger force sensor that is configured for monitoring a finger force signal generated by one or more fingers of the wearer related to the grasping movement task so that the grasp control system **100** can identify the VOI from the movement intention signal and the finger force signal. There may also be one or more external facing sensors **105** for producing additional information signals such as GPS, RFID readers, Wi-Fi signal, etc. that may be used by the grasp control system **100.**

Data storage memory **103** is configured for storing grasp control software, the movement intention signal, and other system information such as various systems settings **107** related to operation of the grasp control system **100** and the powered orthotic **104.** The systems settings **107** may include one or more user-specific settings such as signal gains, signal thresholds, operation speeds, grasp preferences, etc. The system information stored in the data storage memory **103** also may include without limitation device history information, shared performance information, historic control settings, and machine learning data

A grasp control processor **102** including at least one hardware processor is coupled to the data storage memory **103** and configured to execute the grasp control software. The grasp control software includes processor readable instructions to implement a grasp control algorithm for: identifying a volitional operator input for the grasping movement task from the movement intention signal produced by the muscle movement sensor **101.** Operation of the powered orthotic device **104** by the grasp control processor **102** is based on the volitional operator input to perform the grasping movement task as a chain of motion primitives, wherein each motion primitive is a fundamental unit of grasping motion defined along a movement path with a single degree of freedom. Specifically, the grasp control system **100** may operate the powered orthotic device **104** to perform the grasping movement task as chain of motion primitives at a variable speed controlled as a function of the volitional operator input.

The grasp control system **100** implements grasping movement tasks as chains of motion primitives defined by the system, the user or a therapist. The motion primitives describe a simple motion of the powered orthotic **104** with one degree of freedom (DOF), and prescribe a position, velocity, or force in fundamental terms. The motion primitives may be pre-defined, and/or they may be dynamically generated online (on-the-fly) based on sensor inputs such as a motion that maintains spatial position based on a gravitational vector, or maintaining a constant force (which requires some change in position). The motion primitives may be combined in parallel to create complex grasping movement task maneuvers that the powered orthotic **104** can perform. And performing a specific grasping movement task may include undoing a portion of the grasping movement task based on the VOI by performing a portion of the chain of motion primitives in reverse order.

As mentioned above, the motion primitive chains may be pre-defined and stored on the device, or they may be located on a remote server which can be accessed by the grasp control system **100,** or they may be combined online based on branching logic from external or internal sensing inputs. The chains may use directly recorded motions, or they may choose the closest pre-defined motion primitives that match the desired grasping movement task. By scrubbing through the chained motion primitives at a dynamic speed, resulting joint angle velocity commands will depend on both the primitive's desired speed, as well as the VOI, resulting in a shaped motion chain (SMC). The SMC serves as an input to the controllers of the powered orthotic device. The device may impart other control layers on top of the SMC, including but not limited to, closed-loop velocity control, force control or feedback, position limits, kinematic compensations, acceleration limits, and safety thresholds. Volitional Operator Inputs (VOI's) can be used to scrub through the chain of actions, moving forward or reverse through the action instruction set, at speed proportional to measured signal power, current, or voltage.

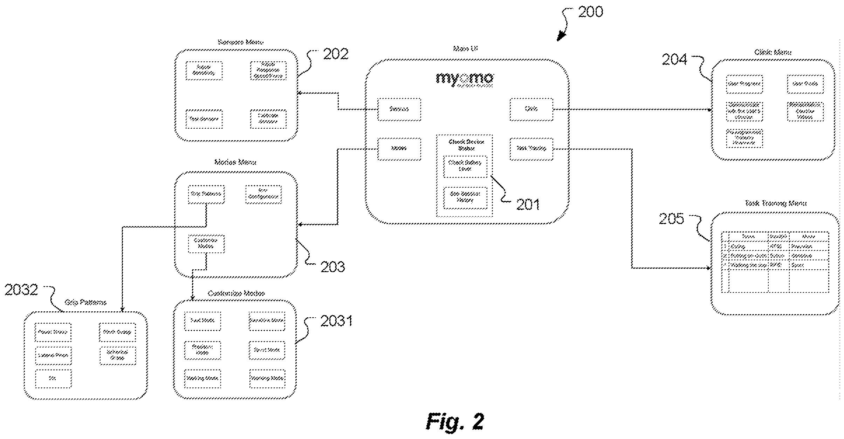

The user can also interact with the grasp control system **100** via a user interface **108** configured to select the system settings and/or operating mode. Figure 2 shows one specific example of a menu architecture for such a user interface **108** that includes a device status section **201** configured to display to the user information such as battery status and session history. Other useful submenus are also available such as a sensor menu **202** to test and calibrate the system input sensors and adjust their sensitivity and response speed and force. A modes menu **203** allows the user to set a specific arm configuration, customize operating modes **2031** (e.g., fast, precision, walking, sensitive, sport, working, etc.), and adjust grip patterns **2032** (e.g., power grasp, pinch grasp, lateral pinch, spherical grasp, etc.). A clinic menu **204** allows monitoring and adjusting of user goals and progress, clinician communication, programming therapy movements and control of rehabilitation exercise videos. A task training menu **205** helps the user program and organize the various assisted movement tasks such as eating, dressing, etc.

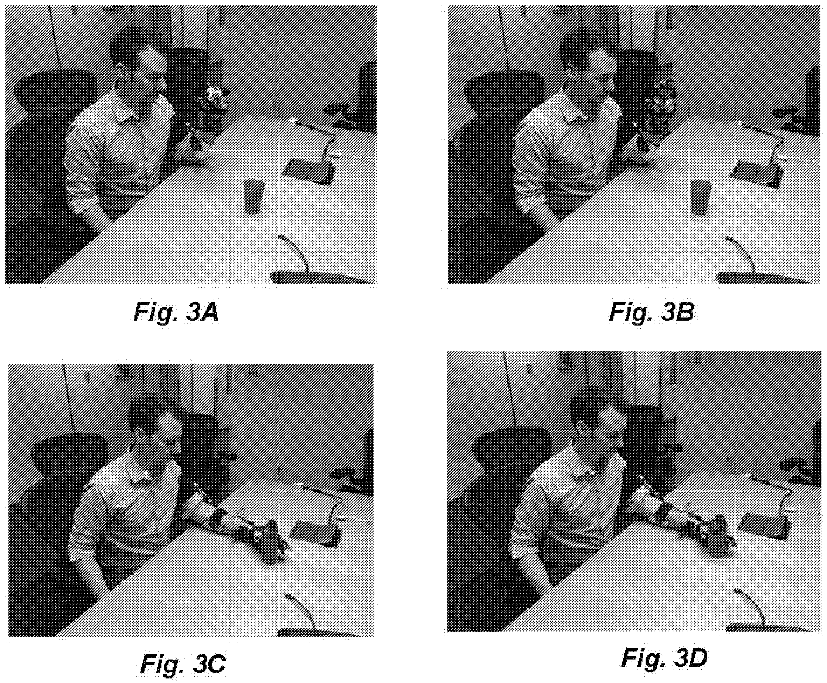

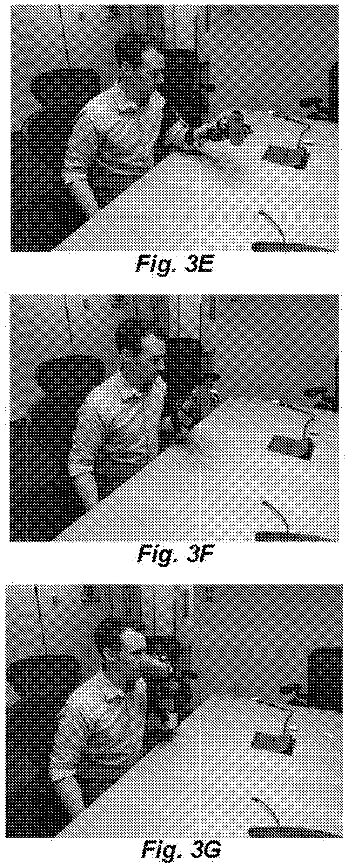

Figures 3A-3G show example photographs of a user fitted with a powered orthotic device that he uses for the specific grasping movement task of lifting a cup for drinking. In Fig. 3A, the user initiates the grasping movement task by a user input such as uttering a voice command to the system. The fingers of the hand then open, Fig. 3B, and the elbow then lowers the open hand down to straddle the cup, Fig. 3C. The next motion primitive to be executed closes the hand around the cup, Fig. 3D. The system then executed the next motion primitive, Fig. 3E, to lift the cup by moving the elbow and wrist in a coordinated manner to keep the cup level. In Fig. 3F, the cup reaches the desired drinking location in front of the user, and the next movement is executed, adjusting the wrist deviation back to neutral to tip the cup towards the mouth, Fig. 3G.

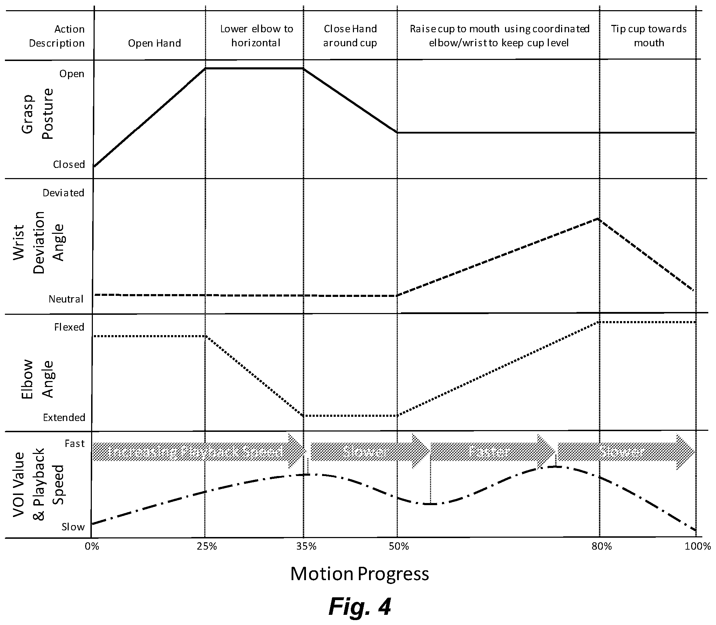

Figure 4 shows a graph of various relevant parameters during such the process shown in Figs. 3A-3G. This illustrates how the grasping movement task of drinking from a cup combines simpler movements using three separate DOF's. Each segment of the different lines for grasp posture, wrist deviation angle, and elbow angle is a separate different motion primitive over time. When combined together in parallel, according to the invention, a complex set grasping movement task actions is created that forms a motion chain.

In specific embodiments, a user could pick up a cup, and also can volitionally slow the motion as the cup grasp is happening, or reverse motion if the grasp attempt missed the cup entirely. They could then speed up the motion as it lifts the cup to decrease overall time to complete a drink.

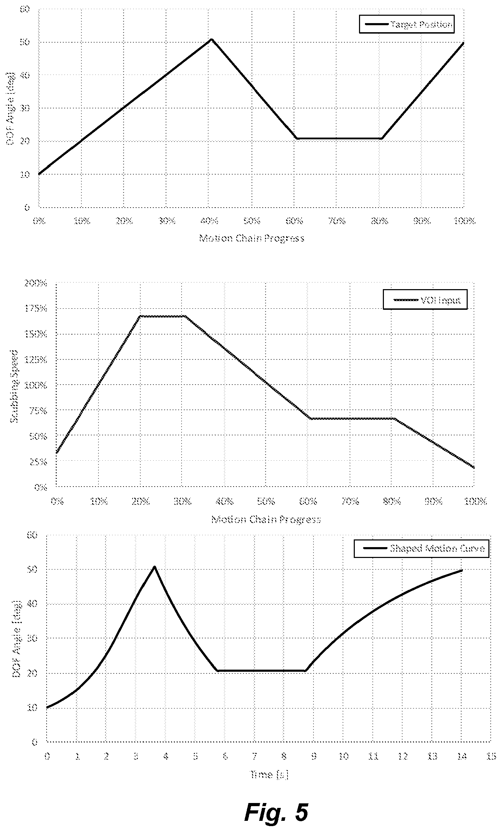

Figure 5 shows an example of how a motion chain is shaped in the case of single direction scrubbing. A single DOF is shown scrubbed at a speed varying between 20% and 170% playback speed. The output graph of position versus time results in variable velocities based on a combination of scrubbing speed and the slope of the target position graph (velocity). Note that the motion chain is defined only in terms of percentage complete, but once played back at a variable rate, the completion time is dependent on both the VOI and the motion chain.

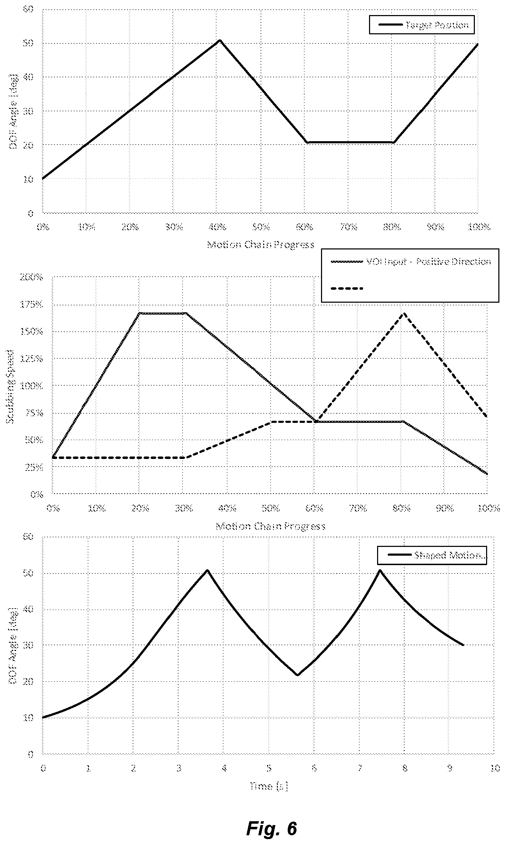

Figure 6 shows a similar set of waveforms for another example with a single DOF scrubbed at a varying speed using two inputs to generate a positive and negative direction VOI. When the positive direction value is higher (left side of the middle waveform), the motion chain is scrubbed in the forward direction at a speed proportional to the VOI value. When the negative direction value is higher (right side of the middle waveform), the motion is scrubbed in a negative direction. The output graph of position versus time results in variable velocities based on a combination of scrubbing speed and the slope of the target position graph (velocity). In this case, 60% of the motion chain is performed, then the direction is reversed, and the motion chain is played back in reverse at a speed proportion to VOI, yielding a somewhat symmetric result.

In one specific embodiment, muscle sensing signals for such grasping movement tasks can be generated by an antagonistic pair of surface electromyography (sEMG) sensors connected to the bicep and tricep of the user and generating the VOIs. Flexing the biceps then generates faster movement through the motion chain, while flexing the triceps causes reverse movement through the motion chain at a speed proportional to signal power.

VOIs may be physiologically related such as for a finger flexor signal being used to perform a finger motion, or they may be physiologically unrelated such as using a pectoral muscle signal to execute a complicated maneuver utilizing coordinated elbow, hand, and wrist movements. For VOI's in a single-input embodiment, a moderate signal level can be considered a stationary threshold level, with lower level signals indicating reverse motion VOIs, and higher level signals indicating forward motion; the greater the absolute value of the signal from the threshold, the faster the speed of motion. An alternative single sensor embodiment would have a zero motion set point near zero signal level, with increasing signal level indicating faster forward motion. When an indication such as a quick twitch is activated, the direction is reversed, with higher signal level indicating faster reverse motion. Instead of a twitch pattern, a voice command or button press by the other hand could also be used to reverse direction. For practicality of signal noise removal, zero motion cannot be at zero signal level, as some signal level will always be measured in the form of noise. Instead, a minimum threshold can be set above the noise floor and any signal below that threshold can be regarded as zero.

Information and/or data may be drawn from other onboard sensors such as for angular position, gravitational vector, localization information, or force/pressure/contact sensors to determine when to transition from one motion primitive to another, or to determine the online shape of the motion primitive. For example, when drinking from a cup, the coordination of ulnar deviation and elbow flexion are linked such that ulnar deviation maintains the cup level as measured by an inertial measurement unit (IMU). Depending on a user's posture, the required angle for specific movements such as pronation, supination, ulnar deviation, etc. may be different during each task execution, so a predefined routine of elbow and wrist position alone would not always yield satisfactory performance. Another example would be that the motion continues to play in the close-grasp direction until force sensors at the hand register sufficient grasp contact with an object. At that point, progression to the next motion primitive is triggered. Logic can also branch and merge, such as closing hand until force is registered OR end of actuator travel is reached.

Besides the muscle sensor arrangements discussed above, the VOI to initiate a motion chain for a given movement task can be generated by some other form of user input such as voice command, pressing a button on the device, scrolling through a list of commands on a phone or tablet, or intelligently selected by the device based on location information (e.g., RFID tags, QR codes or other location tags) or in the case of grasping, using a video camera to identify and classify the object to be grasped.

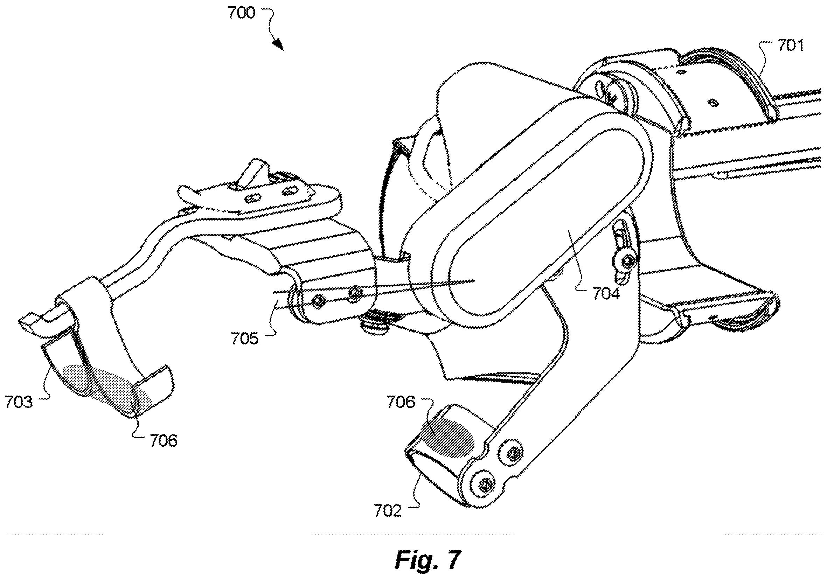

Figure 7 shows an example of the structure of a powered orthotic device **700** suitable for assisting a user with performing a hand task movement according to an embodiment of the present invention. A base section **701** fits over the forearm of the user and includes the muscle movement sensors (not shown, but fitting onto the flexor and extensor muscles of the forearm) that generate the VOIs. Grasp actuator **704** contains the grasp control processor and generates the powered signals to a thumb actuator **702** and finger actuators **703** to assist with their movements during execution of the motion primitives of the motion chains. Force sensors **706** provide feedback signals to the grasp actuator **704** for control of the hand task movements. Device angle **705** indicates the angle of the metacarpophalangeal joint (MCP) where 0 degrees corresponds to the fully open position of the fingers.

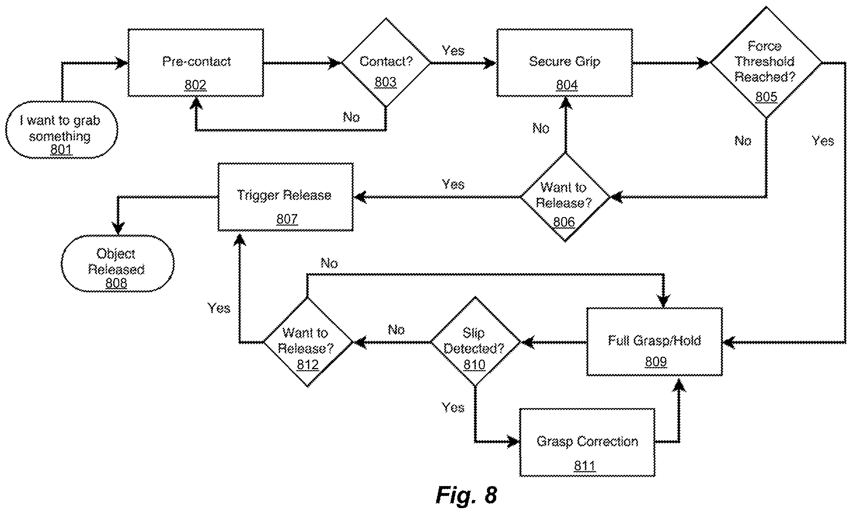

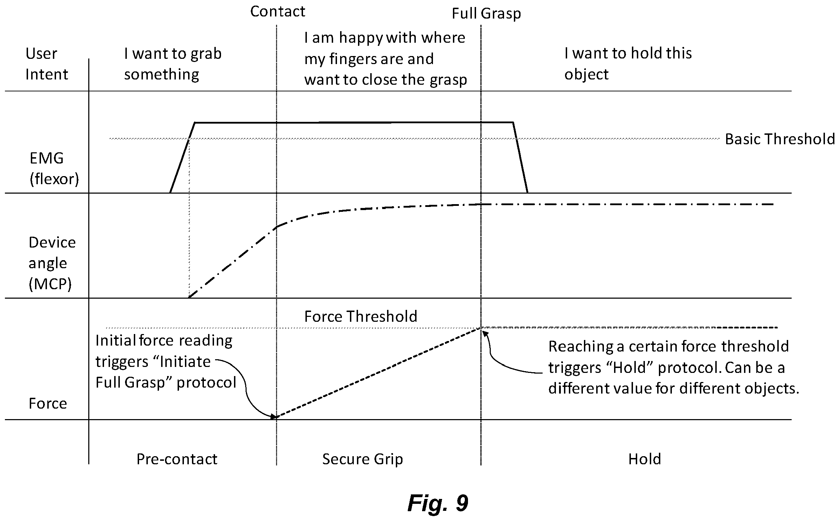

Figure 8 is grasp-release flowchart showing various logical steps in a grasp control process using motion chains according to an embodiment of the present invention, specifically showing grasping, holding, and releasing an object. When the user first determines: "I want to grab something", step **801,** the user generates an initial VOI by bringing their flexor EMG signal above a tunable "basic threshold" to start the initial precontact movement of the powered orthotic device, step **802.** Once contact is made with the object, step **803,** the finger force sensors will read an initial non-zero force value which changes the grasp mode from "pre-contact" to "secure grip", step **804.** In "secure grip" mode, as long as the flexor EMG signal is above the threshold, the rate that the fingers close (device angle slope) is driven by the force sensors. Once a certain force threshold is reached by the force sensors, step **805,** the grasp mode changes from "secure grip" mode to "hold" mode, step **809.** In "hold" mode, the user can relax their flexor EMG signal below the basic threshold and the device will maintain its grip on the object. Various example waveforms associated with this basic precontact-secure-hold sequence are shown in Figure 9.

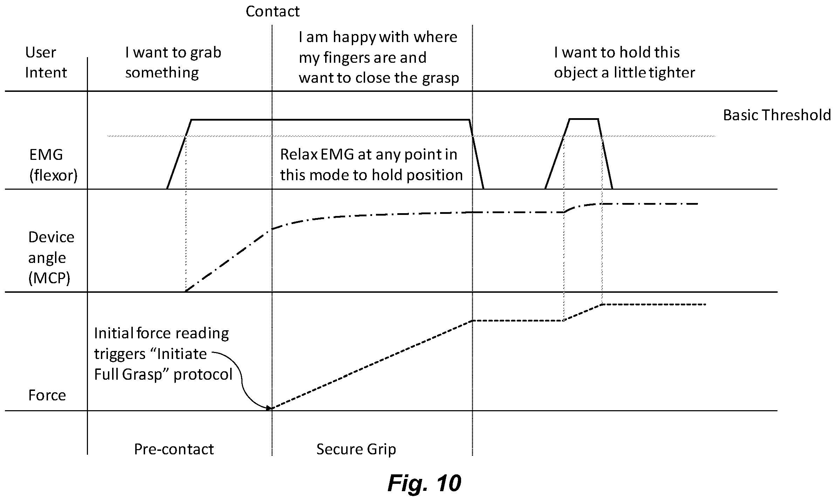

A hold/ratcheting mode can also be provided where, once the user is in "secure grip" mode, step **804,** they can relax their flexor EMG signal at any point to hold position, or the user can raise their flexor EMG signal above the basic threshold once relaxed to continue to increase the device angle and force holding the object. Various example waveforms associated with this basic precontact-secure-hold/ratcheting sequence are shown in Figure 10.

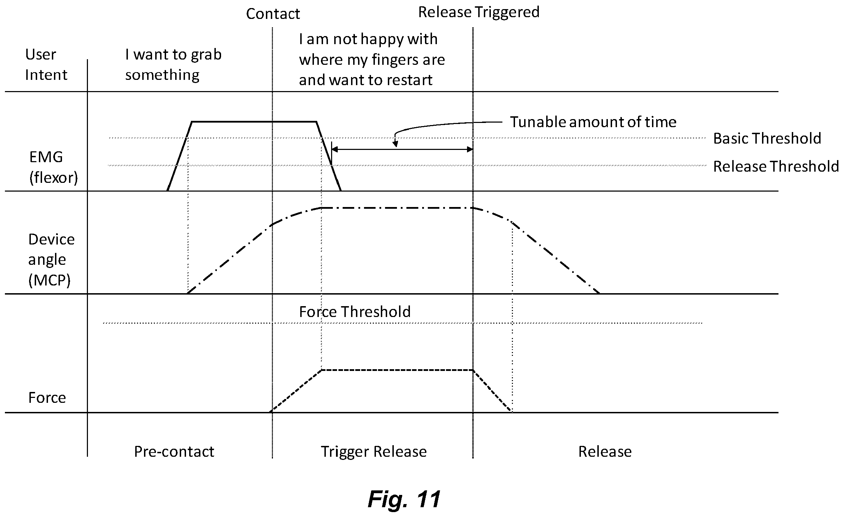

Rather than smoothly progressing from the secure grip mode, step **804,** to the hold mode, step **809,** something may go wrong in the process of securing the grip such that the user wants to release the object. In an embodiment with a single flexor muscle sensor, at a certain point in the process when the user is not happy with securing their grasp, they then release their flexor muscle sensor signal until it falls below a release threshold, step **806,** and so a release mode is triggered, step **807,** and the object is released during which the rate that the fingers open (device angle slope) is driven by the force sensors. Figure 11 shows various example waveforms associated with this single sensor precontact-trigger release-release process.

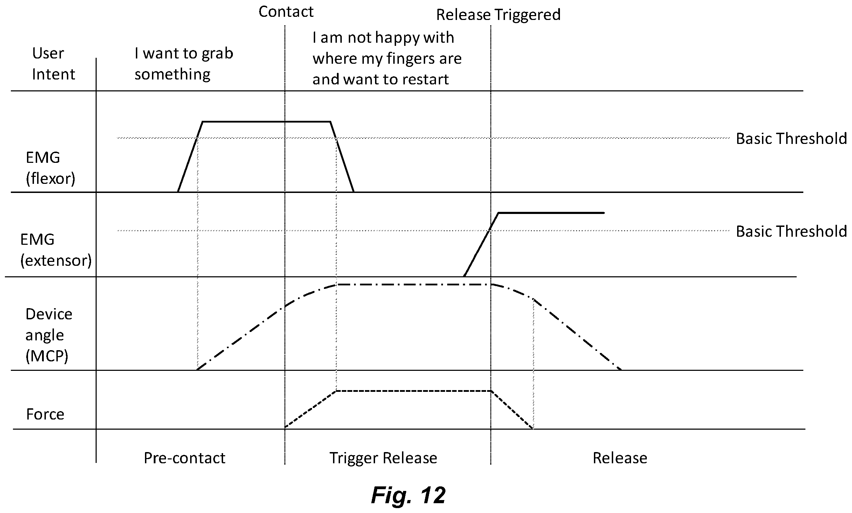

In a two sensor embodiment with both flexor and extensor muscle sensor signals available for VOI, the user initiates movement, step **802** by bringing their flexor sensor signal above a tunable basic threshold until contact is made with the object, step **803.** But at some point, the user is not happy with securing their grasp, step **806,** and releases their flexor sensor signal and raises their extensor sensor signal above a tunable basic threshold, step **806,** to trigger the release mode, step **807,** and the object is released, step **808,** as long as the user maintains their extensor EMG signal above the basic threshold. When the object is released, again the rate that the fingers open (device angle slope) is driven by the force sensors. Figure 12 shows various example waveforms associated with this two sensor precontact-trigger release-release process.

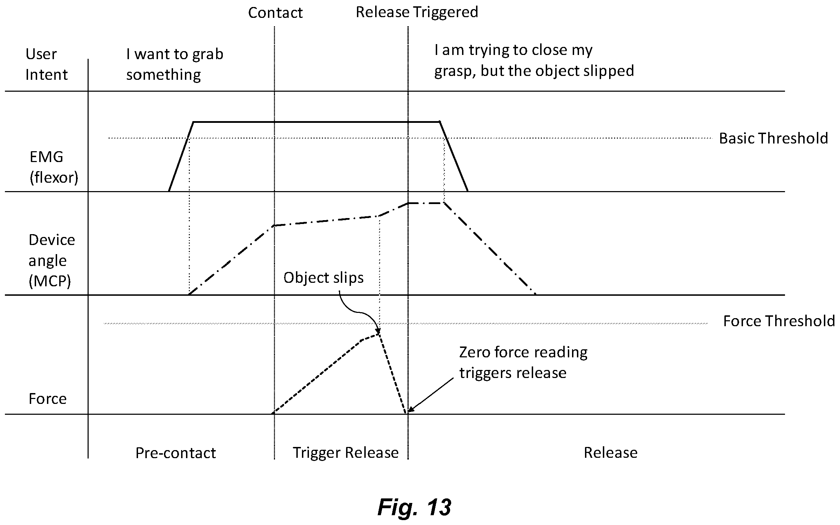

Rather than a conscious decision to release the object, the object may inadvertently slip so that the force sensors generate a zero force signal that triggers the release mode. Specifically, once in the full grasp/hold mode, step **809,** if a slip is detected, step **810,** a grasp correction may be attempted, step **811,** or otherwise, the trigger release mode is entered, step **807,** and the object is released, step **808.** Figure 13 shows various example waveforms for this slipping for a single flexor sensor which when relaxed below the basic threshold in release mode opens up the user's grasp (bringing the device angle back down to zero). In the event of having two muscle sensors, raising the extensor sensor signal above the basic threshold would also open up the user's grasp.

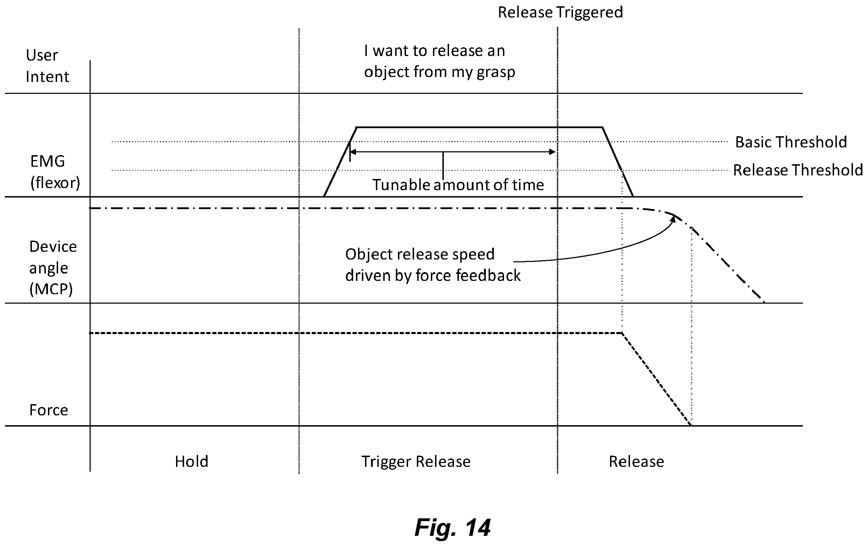

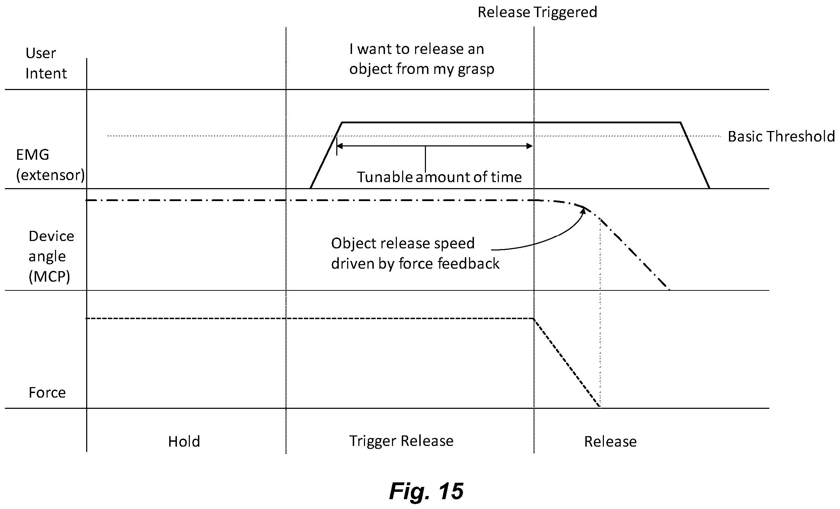

In the full grasp/hold mode, step **809,** the user holds the object with no significant VOI signal. Once the user wants to release the object, he/she increases the VOI signal above the basic threshold for a tunable amount of time, step **812,** until the trigger release mode is entered, step **807.** Once in release mode, the user can release the object, step **808,** by relaxing VOI signal below the release threshold and the fingers will open up at a rate driven by the force sensors until the force sensors do not read any force at which the fingers will open up at a constant rate. Figure 14 shows various example waveforms for this slipping for a single flexor sensor, and Figure 15 shows similar waveforms for a dual sensor embodiment.

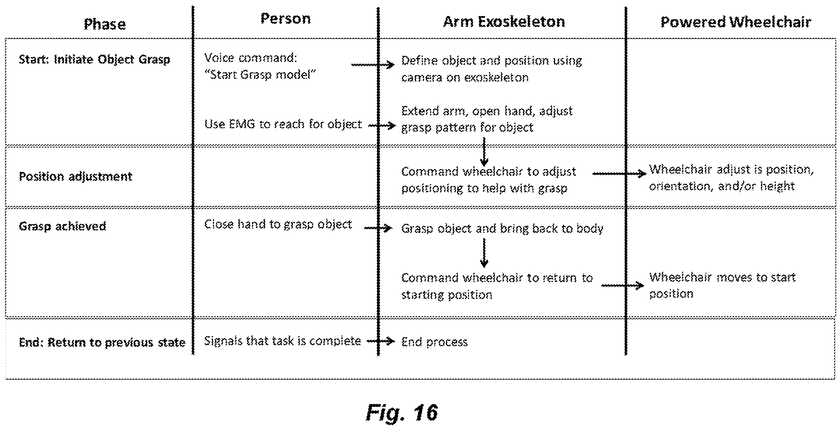

A powered orthotic device and grasp control system such as described above can provide enhance functionality for other external devices in order to complete an action the other device is unable to achieve by itself; that is, it may be useful to coordinate multiple different devices to accomplish some grasping tasks. For example, such an arrangement can help a user sitting in a wheelchair ("other external device") to grasping an object that is currently out of reach on a table. Figure 16 illustrates operation in such a scenario where the powered orthotic device is referred to as an "Arm Exoskeleton", which is able to coordinate its operation with a powered wheelchair to complete more complicated or larger movement tasks than could be done by the user with just one of the devices. Specifically, the Arm Exoskeleton and the Powered Wheelchair may be configured in a master-slave arrangement where explicit commands are sent from the master device to the slave device telling the slave device what to do to perform the movement task being controlled by the master device.

New grasps and motion chains can be learned and acquired as needed based on the situation in real time. Examples of such new tasks that might arises in normal life might include grasping a new kind of object like a heavy boot, operating a new handicap access button, using the device to play a new sport like swinging a golf club, or even as simple as adjusting the grip size for a new coffee mug. Such new task scenarios can be triggered based on, for example, a camera-based image classifier, by selecting new tasks from a menu, or by an audio download command. In addition or alternatively, the grasp control system may regularly or on-demand connect to a remote server that provides it with new behaviors/daily updates.

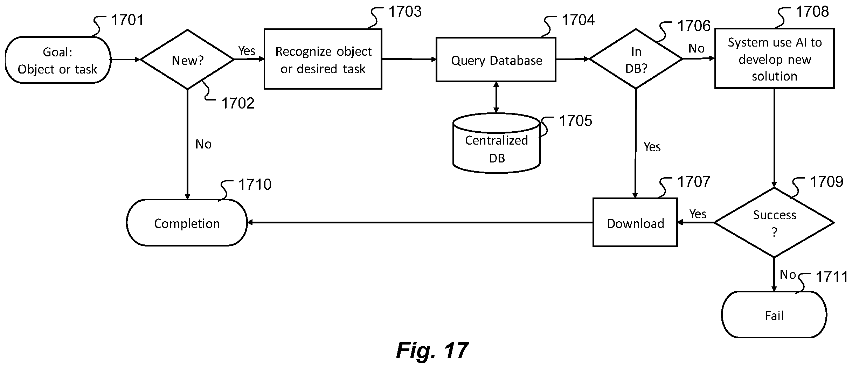

Figure 17 shows one specific arrangement for acquiring such new task motion chains. When the system identifies a task or goal, step **1701,** and determines that this task is not presently defined, step **1702,** it then initially defines that task in real time, and accesses a remote query database, step **1704,** to obtain instructions for the new task from a central database **1705.** If instructions for the new task are present in central database, step **1706,** the system downloads the instructions for the new task, step **1707,** which can then be completed, step **1710.** If instructions for the new task are not present in central database at step **1706,** the system can attempt to develop a new solution, step **1708.** Such developing of the motion chains for a new solution can be handled locally on the device, or remotely at a central server, or by combination and coordination of both local and remote resources. If that succeeds, step **1709,** then the system downloads the new instructions for the new task, step **1707,** which can then be completed, step **1710.** If not, the routine ends in failure, step **1711,** having failed to obtain the instructions for the new task. Such new task solutions that are developed may also be uploaded back to the central database to be available for other users (e.g., pick up the same style cup, etc.)

Embodiments of the invention may be implemented in part in any conventional computer programming language such as VHDL, SystemC, Verilog, ASM, etc. Alternative embodiments of the invention may be implemented as pre-programmed hardware elements, other related components, or as a combination of hardware and software components.

Embodiments can be implemented in part as a computer program product for use with a computer system. Such implementation may include a series of computer instructions fixed either on a tangible medium, such as a computer readable medium (*e*.*g*., a diskette, CD-ROM, ROM, or fixed disk) or transmittable to a computer system, via a modem or other interface device, such as a communications adapter connected to a network over a medium. The medium may be either a tangible medium (*e*.*g*., optical or analog communications lines) or a medium implemented with wireless techniques (*e*.*g*., microwave, infrared or other transmission techniques). The series of computer instructions embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (*e*.*g*., shrink wrapped software), preloaded with a computer system (*e*.*g*., on system ROM or fixed disk), or distributed from a server or electronic bulletin board over the network (*e*.*g*., the Internet or World Wide Web). Of course, some embodiments of the invention may be implemented as a combination of both software (*e*.*g*., a computer program product) and hardware. Still other embodiments of the invention are implemented as entirely hardware, or entirely software (*e*.*g*., a computer program product).

Although various exemplary embodiments of the invention have been disclosed, it should be apparent to those skilled in the art that various changes and modifications can be made which will achieve some of the advantages of the invention without departing from the true scope of the invention.
their flexor muscle sensor signal until it falls below a release threshold, step **806,** and so a release mode is triggered, step **807,** and the object is released during which the rate that the fingers open (device angle slope) is driven by the force sensors. Figure 11 shows various example waveforms associated with this single sensor precontact-trigger release-release process.

In a two sensor embodiment with both flexor and extensor muscle sensor signals available for VOI, the user initiates movement, step **802** by bringing their flexor sensor signal above a tunable basic threshold until contact is made with the object, step **803.** But at some point, the user is not happy with securing their grasp, step **806,** and releases their flexor sensor signal and raises their extensor sensor signal above a tunable basic threshold, step **806,** to trigger the release mode, step **807,** and the object is released, step **808,** as long as the user maintains their extensor EMG signal above the basic threshold. When the object is released, again the rate that the fingers open (device angle slope) is driven by the force sensors. Figure 12 shows various example waveforms associated with this two sensor precontact-trigger release-release process.

Rather than a conscious decision to release the object, the object may inadvertently slip so that the force sensors generate a zero force signal that triggers the release mode. Specifically, once in the full grasp/hold mode, step **809,** if a slip is detected, step **810,** a grasp correction may be attempted, step **811,** or otherwise, the trigger release mode is entered, step **807,** and the object is released, step **808.** Figure 13 shows various example waveforms for this slipping for a single flexor sensor which when relaxed below the basic threshold in release mode opens up the user's grasp (bringing the device angle back down to zero). In the event of having two muscle sensors, raising the extensor sensor signal above the basic threshold would also open up the user's grasp.

In the full grasp/hold mode, step **809,** the user holds the object with no significant VOI signal. Once the user wants to release the object, he/she increases the VOI signal above the basic threshold for a tunable amount of time, step **812,** until the trigger release mode is entered, step **807.** Once in release mode, the user can release the object, step **808,** by relaxing VOI signal below the release threshold and the fingers will open up at a rate driven by the force sensors until the force sensors do not read any force at which the fingers will open up at a constant rate. Figure 14 shows various example waveforms for this slipping for a single flexor sensor, and Figure 15 shows similar waveforms for a dual sensor embodiment.

A powered orthotic device and grasp control system such as described above can provide enhance functionality for other external devices in order to complete an action the other device is unable to achieve by itself; that is, it may be useful to coordinate multiple different devices to accomplish some grasping tasks. For example, such an arrangement can help a user sitting in a wheelchair ("other external device") to grasping an object that is currently out of reach on a table. Figure 16 illustrates operation in such a scenario where the powered orthotic device is referred to as an "Arm Exoskeleton", which is able to coordinate its operation with a powered wheelchair to complete more complicated or larger movement tasks than could be done by the user with just one of the devices. Specifically, the Arm Exoskeleton and the Powered Wheelchair may be configured in a master-slave arrangement where explicit commands are sent from the master device to the slave device telling the slave device what to do to perform the movement task being controlled by the master device.

New grasps and motion chains can be learned and acquired as needed based on the situation in real time. Examples of such new tasks that might arises in normal life might include grasping a new kind of object like a heavy boot, operating a new handicap access button, using the device to play a new sport like swinging a golf club, or even as simple as adjusting the grip size for a new coffee mug. Such new task scenarios can be triggered based on, for example, a camera-based image classifier, by selecting new tasks from a menu, or by an audio download command. In addition or alternatively, the grasp control system may regularly or on-demand connect to a remote server that provides it with new behaviors/daily updates.

Figure 17 shows one specific arrangement for acquiring such new task motion chains. When the system identifies a task or goal, step **1701,** and determines that this task is not presently defined, step **1702,** it then initially defines that task in real time, and accesses a remote query database, step **1704,** to obtain instructions for the new task from a central database **1705.** If instructions for the new task are present in central database, step **1706,** the system downloads the instructions for the new task, step **1707,** which can then be completed, step **1710.** If instructions for the new task are not present in central database at step **1706,** the system can attempt to develop a new solution, step **1708.** Such developing of the motion chains for a new solution can be handled locally on the device, or remotely at a central server, or by combination and coordination of both local and remote resources. If that succeeds, step **1709,** then the system downloads the new instructions for the new task, step **1707,** which can then be completed, step **1710.** If not, the routine ends in failure, step **1711,** having failed to obtain the instructions for the new task. Such new task solutions that are developed may also be uploaded back to the central database to be available for other users (e.g., pick up the same style cup, etc.)

Embodiments of the invention may be implemented in part in any conventional computer programming language such as VHDL, SystemC, Verilog, ASM, etc. Alternative embodiments of the invention may be implemented as pre-programmed hardware elements, other related components, or as a combination of hardware and software components.

Embodiments can be implemented in part as a computer program product for use with a computer system. Such implementation may include a series of computer instructions fixed either on a tangible medium, such as a computer readable medium (*e*.*g*., a diskette, CD-ROM, ROM, or fixed disk) or transmittable to a computer system, via a modem or other interface device, such as a communications adapter connected to a network over a medium. The medium may be either a tangible medium (*e*.*g*., optical or analog communications lines) or a medium implemented with wireless techniques (*e*.*g*., microwave, infrared or other transmission techniques). The series of computer instructions embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (*e*.*g*., shrink wrapped software), preloaded with a computer system (*e*.*g*., on system ROM or fixed disk), or distributed from a server or electronic bulletin board over the network (*e*.*g*., the Internet or World Wide Web). Of course, some embodiments of the invention may be implemented as a combination of both software (*e.g*., a computer program product) and hardware. Still other embodiments of the invention are implemented as entirely hardware, or entirely software (*e*.*g*., a computer program product).

Although various exemplary embodiments of the invention have been disclosed, it should be apparent to those skilled in the art that various changes and modifications can be made which will achieve some of the advantages of the invention without departing from the true scope of the invention.

## Claims

1. A computer-implemented method employing at least one hardware implemented computer processor (102) for controlling a grasp control system (100) to assist an operator with a grasping movement task, the method comprising:
operating the at least one hardware processor (102) to execute program instructions for:
monitoring movement intention signal of a grasping movement muscle of the operator;
identifying a volitional operator input for the grasping movement task from the movement intention signal;
operating a powered orthotic device (104, 700) based on the volitional operator input to perform the grasping movement task as a chain of motion primitives, wherein each motion primitive is a fundamental unit of grasping motion defined along a movement path with a single degree of freedom, the motion primitives being configured to be combined in parallel to form the chain of motion primitives.

2. The method of claim 1, wherein operating the powered orthotic device (104, 700) includes performing the grasping movement task as chain of motion primitives at a variable speed controlled as a function of the volitional operator input.

3. The method of claim 1, further comprising:
monitoring a second movement intention signal of a second grasping movement muscle of the wearer, wherein the volitional operator input is identified from both movement intention signals,
wherein the grasping movement muscles monitored by the movement intention signals are preferably antagonistic muscles.

4. The method of claim 1, wherein performing the grasping movement task further comprises:
undoing a portion of the grasping movement task based on the volitional operator input by performing a portion of the chain of motion primitives in reverse order.

5. The method of claim 1, further comprising:
monitoring a finger force signal generated by one or more fingers of the wearer related to the grasping movement task, wherein the volitional operator input is identified from the movement intention signal and the finger force signal.

6. The method of claim 1, wherein the chain of motion primitives creates grasping motion with at least two degrees of freedom.

7. The method of claim 1, wherein the chain of motion primitives are one of predefined system chains, user defined chains, and dynamically defined by the user, or wherein the movement intention signal is an electromyography, EMG, signal.

8. A computer-implemented grasp control system (100) for assisting an operator with a grasping movement task, the system comprising:
a muscle movement sensor (101) configured for monitoring a grasping movement muscle of the operator to produce a movement intention signal;
a powered orthotic device (104, 700) configured for assisting grasping motion of the operator;
data storage memory (103) configured for storing grasp control software, the movement intention signal, and other system information;
a grasp control processor (102) including at least one hardware processor coupled to the data storage memory (103) and configured to execute the grasp control software, wherein the grasp control software includes processor readable instructions to implement a grasp control algorithm for:
identifying a volitional operator input for the grasping movement task from the movement intention signal;
operating the powered orthotic device (104, 700) based on the volitional operator input to perform the grasping movement task as a chain of motion primitives, wherein each motion primitive is a fundamental unit of grasping motion defined along a movement path with a single degree of freedom, the motion primitives being configured to be combined in parallel to form the chain of motion primitives.

9. The grasp control system (100) according to claim 8, wherein the grasp control algorithm operates the powered orthotic device (104, 700) to perform the grasping movement task as chain of motion primitives at a variable speed controlled as a function of the volitional operator input.

10. The grasp control system (100) of claim 8, further comprising:
a second muscle movement sensor configured for monitoring a second grasping movement muscle of the operator to produce a second movement intention signal, wherein the grasp control algorithm identifies the volitional operator input from both movement intention signals,
wherein the grasping movement muscles are preferably antagonistic muscles.

11. The grasp control system (100) of claim 8, wherein performing the grasping movement task further comprises:
undoing a portion of the grasping movement task based on the volitional operator input by performing a portion of the chain of motion primitives in reverse order.

12. The grasp control system (100) of claim 8, further comprising:
a finger force sensor (106, 706) configured for monitoring a finger force signal generated by one or more fingers of the wearer related to the grasping movement task, wherein the grasp control algorithm identifies the volitional operator input from the movement intention signal and the finger force signal.

13. The grasp control system (100) of claim 8, wherein the chain of motion primitives creates grasping motion with at least two degrees of freedom.

14. The grasp control system (100) of claim 8, wherein the chain of motion primitives are one of predefined system chains, user defined chains, and dynamically defined by the user.

15. The grasp control system (100) of claim 8, wherein the muscle movement sensor (101) is an electromyography, EMG, signal sensor.

## Patentansprüche

1. Computerimplementiertes Verfahren, das mindestens einen hardwareimplementierten Computerprozessor (102) zum Steuern eines Greifsteuersystems (100) verwendet, um einen Bediener bei einer Greifbewegungsaufgabe zu unterstützen, wobei das Verfahren Folgendes umfasst:
Betreiben des mindestens einen Hardwareprozessors (102), um Programmanweisungen auszuführen zum:
Überwachen eines Bewegungsintentionssignals eines Greifbewegungsmuskels des Bedieners;
Identifizieren einer willentlichen Bedienereingabe für die Greifbewegungsaufgabe aus dem Bewegungsintentionssignal;
Betreiben einer angetriebenen orthetischen Vorrichtung (104, 700) basierend auf der willentlichen Bedienereingabe, um die Greifbewegungsaufgabe als eine Kette von Bewegungsprimitiven durchzuführen, wobei jedes Bewegungsprimitiv eine Grundeinheit einer Greifbewegung ist, die entlang eines Bewegungspfads mit einem einzigen Freiheitsgrad definiert ist, wobei die Bewegungsprimitive konfiguriert sind, um parallel kombiniert zu werden, um die Kette von Bewegungsprimitiven zu bilden.

2. Verfahren nach Anspruch 1, wobei das Betreiben der angetriebenen orthetischen Vorrichtung (104, 700) das Durchführen der Greifbewegungsaufgabe als eine Kette von Bewegungsprimitiven mit einer variablen Geschwindigkeit beinhaltet, die als eine Funktion der willentlichen Bedienereingabe gesteuert wird.

3. Verfahren nach Anspruch 1, ferner umfassend:
Überwachen eines zweiten Bewegungsintentionssignals eines zweiten Greifbewegungsmuskels des Trägers, wobei die willentliche Bedienereingabe aus beiden Bewegungsintentionssignalen identifiziert wird,
wobei die Greifbewegungsmuskeln, die durch die Bewegungsintentionssignale überwacht werden, vorzugsweise antagonistische Muskeln sind.

4. Verfahren nach Anspruch 1, wobei das Durchführen der Greifbewegungsaufgabe ferner Folgendes umfasst:
Rückgängigmachen eines Teils der Greifbewegungsaufgabe basierend auf der willentlichen Bedienereingabe durch Durchführen eines Teils der Kette von Bewegungsprimitiven in umgekehrter Reihenfolge.

5. Verfahren nach Anspruch 1, ferner umfassend:
Überwachen eines Fingerkraftsignals, das durch einen oder mehrere Finger des Trägers in Bezug auf die Greifbewegungsaufgabe erzeugt wird, wobei die willentliche Bedienereingabe aus dem Bewegungsintentionssignal und dem Fingerkraftsignal identifiziert wird.

6. Verfahren nach Anspruch 1, wobei die Kette von Bewegungsprimitiven eine Greifbewegung mit mindestens zwei Freiheitsgraden erzeugt.

7. Verfahren nach Anspruch 1, wobei die Kette von Bewegungsprimitiven eines von vordefinierten Systemketten, benutzerdefinierten Ketten und dynamisch durch den Benutzer definiert ist, oder wobei das Bewegungsintentionssignal ein Elektromyographie-, EMG-, Signal ist.

8. Computerimplementiertes Greifsteuersystem (100) zum Unterstützen eines Bedieners bei einer Greifbewegungsaufgabe, wobei das System Folgendes umfasst:
einen Muskelbewegungssensor (101), der zum Überwachen eines Greifbewegungsmuskels des Bedieners konfiguriert ist, um ein Bewegungsintentionssignal zu erzeugen;
eine angetriebene orthetische Vorrichtung (104, 700), die zum Unterstützen einer Greifbewegung des Bedieners konfiguriert ist;
einen Datenspeicher (103), der zum Speichern von Greifsteuersoftware, des Bewegungsintentionssignals und anderer Systeminformationen konfiguriert ist;
einen Greifsteuerprozessor (102), der mindestens einen Hardwareprozessor beinhaltet, der mit dem Datenspeicher (103) gekoppelt und konfiguriert ist, um die Greifsteuersoftware auszuführen, wobei die Greifsteuersoftware prozessorlesbare Anweisungen beinhaltet, um einen Greifsteueralgorithmus zu implementieren zum:
Identifizieren einer willentlichen Bedienereingabe für die Greifbewegungsaufgabe aus dem Bewegungsintentionssignal;
Betreiben der angetriebenen orthetischen Vorrichtung (104, 700) basierend auf der willentlichen Bedienereingabe, um die Greifbewegungsaufgabe als eine Kette von Bewegungsprimitiven durchzuführen, wobei jedes Bewegungsprimitiv eine Grundeinheit einer Greifbewegung ist, die entlang eines Bewegungspfads mit einem einzigen Freiheitsgrad definiert ist, wobei die Bewegungsprimitive konfiguriert sind, um parallel kombiniert zu werden, um die Kette von Bewegungsprimitiven zu bilden.

9. Greifsteuersystem (100) nach Anspruch 8, wobei der Greifsteueralgorithmus die angetriebene orthetische Vorrichtung (104, 700) betreibt, um die Greifbewegungsaufgabe als eine Kette von Bewegungsprimitiven mit einer variablen Geschwindigkeit durchzuführen, die als eine Funktion der willentlichen Bedienereingabe gesteuert wird.

10. Greifsteuersystem (100) nach Anspruch 8, ferner umfassend:
einen zweiten Muskelbewegungssensor, der zum Überwachen eines zweiten Greifbewegungsmuskels des Bedieners konfiguriert ist, um ein zweites Bewegungsintentionssignal zu erzeugen, wobei der Greifsteueralgorithmus die willentliche Bedienereingabe aus beiden Bewegungsintentionssignalen identifiziert,
wobei die Greifbewegungsmuskeln vorzugsweise antagonistische Muskeln sind.

11. Greifsteuersystem (100) nach Anspruch 8, wobei das Durchführen der Greifbewegungsaufgabe ferner Folgendes umfasst:
Rückgängigmachen eines Teils der Greifbewegungsaufgabe basierend auf der willentlichen Bedienereingabe durch Durchführen eines Teils der Kette von Bewegungsprimitiven in umgekehrter Reihenfolge.

12. Greifsteuersystem (100) nach Anspruch 8, ferner umfassend:
einen Fingerkraftsensor (106, 706), der zum Überwachen eines Fingerkraftsignals konfiguriert ist, das durch einen oder mehrere Finger des Trägers in Bezug auf die Greifbewegungsaufgabe erzeugt wird, wobei der Greifsteueralgorithmus die willentliche Bedienereingabe aus dem Bewegungsintentionssignal und dem Fingerkraftsignal identifiziert.

13. Greifsteuersystem (100) nach Anspruch 8, wobei die Kette von Bewegungsprimitiven eine Greifbewegung mit mindestens zwei Freiheitsgraden erzeugt.

14. Greifsteuersystem (100) nach Anspruch 8, wobei die Kette von Bewegungsprimitiven eines von vordefinierten Systemketten, benutzerdefinierten Ketten und dynamisch durch den Benutzer definiert ist.

15. Greifsteuersystem (100) nach Anspruch 8, wobei der Muskelbewegungssensor (101) ein Elektromyographie-, EMG-, Signalsensor ist.

## Revendications

1. Procédé mis en oeuvre par ordinateur utilisant au moins un processeur mis en oeuvre par matériel (102) pour commander un système de commande de préhension (100) pour assister un opérateur pour une tâche de mouvement de préhension, le procédé comprenant :
l'actionnement de l'au moins un processeur matériel (102) pour exécuter des instructions de programme pour :
surveiller un signal d'intention de mouvement d'un muscle de mouvement de préhension de l'opérateur ;
l'identification d'une entrée d'opérateur volitif pour la tâche de mouvement de préhension à partir du signal d'intention de mouvement ;
l'actionnement d'un dispositif orthétique motorisé (104, 700) sur la base de l'entrée d'opérateur volitif pour effectuer la tâche de mouvement de préhension sous la forme d'une chaîne de primitives de mouvement, dans lequel chaque primitive de mouvement est une unité fondamentale de mouvement de préhension définie le long d'un trajet de mouvement avec un degré de liberté unique, les primitives de mouvement étant configurées pour être combinées en parallèle pour former la chaîne de primitives de mouvement.

2. Procédé selon la revendication 1, dans lequel l'actionnement d'un dispositif orthétique motorisé (104, 700) comprend la réalisation de la tâche de mouvement de préhension en tant que chaîne de primitives de mouvement à une vitesse variable régulée en fonction de l'entrée d'opérateur volitif.

3. Procédé selon la revendication 1, comprenant en outre :
la surveillance d'un deuxième signal d'intention de mouvement d'un deuxième muscle de mouvement de préhension du porteur, dans lequel l'entrée d'opérateur volitif est identifiée à partir des signaux d'intention de mouvement,
dans lequel les muscles de mouvement de préhension surveillés par le signaux d'intention de mouvement sont de préférence des muscles antagonistes.

4. Procédé selon la revendication 1, dans lequel la conduite de la tâche de mouvement de préhension comprend en outre :
l'annulation d'une partie de la tâche de mouvement de préhension sur la base de l'entrée d'opérateur volitif par exécution d'une partie de la chaîne de primitives de mouvement dans l'ordre inverse.

5. Procédé selon la revendication 1, comprenant en outre :
la surveillance d'un signal de force de doigt généré par un ou plusieurs doigts du porteur associé à la tâche de mouvement de préhension, dans lequel l'entrée d'opérateur volitif est identifiée à partir du signal d'intention de mouvement et du signal de force de doigt.

6. Procédé selon la revendication 1, dans lequel la chaîne de primitives de mouvement crée un mouvement de préhension avec au moins deux degrés de liberté.

7. Procédé selon la revendication 1, dans lequel la chaîne de primitives de mouvement sont l'une parmi des chaînes de système prédéfinies, des chaînes définies par l'utilisateur, et définies dynamiquement par l'utilisateur, ou dans lequel le signal d'intention de mouvement est un signal d'électromyographie, EMG.

8. Système de commande de préhension mis en oeuvre par ordinateur (100) pour aider un opérateur pour une tâche de mouvement de préhension, le système comprenant :
un capteur de mouvement musculaire (101) configuré pour surveiller un muscle de mouvement de préhension de l'opérateur pour produire un signal d'intention de mouvement ;
un dispositif orthétique motorisé (104, 700) configuré pour faciliter un mouvement de préhension de l'opérateur ;
une mémoire ode stockage de données (103) configurée pour stocker un logiciel de commande de préhension, le signal d'intention de mouvement, et d'autres informations de système ;
un processeur de commande de préhension (102) comprenant au moins un processeur matériel couplé à la mémoire de stockage de données (103) et configuré pour exécuter le logiciel de commande de préhension, dans lequel le logiciel de commande de préhension comprend des instructions lisibles par processeur pour mettre en oeuvre un algorithme de commande de préhension pour :
identifier une entrée d'opérateur volitif pour la tâche de mouvement de préhension à partir du signal d'intention de mouvement ;
actionner le dispositif orthétique motorisé (104, 700) sur la base de l'entrée d'opérateur volitif pour effectuer la tâche de mouvement de préhension sous la forme d'une chaîne de primitives de mouvement, dans lequel chaque primitive de mouvement est une unité fondamentale de mouvement de préhension définie le long d'un trajet de mouvement avec un seul degré de liberté, les primitives de mouvement étant configurées pour être combinées en parallèle pour former la chaîne de primitives de mouvement.

9. Système de commande de préhension (100) selon la revendication 8, dans lequel l'algorithme de commande de préhension actionne le dispositif orthétique motorisé (104, 700) pour effectuer la tâche de mouvement de préhension sous la forme d'une chaîne de primitives de mouvement à une vitesse variable régulée en fonction de l'entrée d'opérateur volitif.

10. Système de commande de préhension (100) selon la revendication 8, comprenant en outre :
un deuxième capteur de mouvement musculaire configuré pour surveiller un deuxième muscle de mouvement de préhension de l'opérateur pour produire un deuxième signal d'intention de mouvement, dans lequel l'algorithme de commande de préhension identifie l'entrée d'opérateur volitif à partir des deux signaux d'intention de mouvement,
dans lequel les muscles de mouvement de préhension sont de préférence des muscles antagonistes.

11. Système de commande de préhension (100) selon la revendication 8, dans lequel l'exécution de la tâche de mouvement de préhension comprend en outre :
l'annulation d'une partie de la tâche de mouvement de préhension sur la base de l'entrée d'opérateur volitif par exécution d'une partie de la chaîne de primitives de mouvement dans l'ordre inverse.

12. Système de commande de préhension (100) selon la revendication 8, comprenant en outre :
un capteur de force de doigt (106, 706) configuré pour surveiller un signal de force de doigt généré par un ou plusieurs doigts de l'utilisateur associé à la tâche de mouvement de préhension, dans lequel l'algorithme de commande de préhension identifie l'entrée d'opérateur volitif à partir du signal d'intention de mouvement et du signal de force de doigt.

13. Système de commande de préhension (100) selon la revendication 8, dans lequel la chaîne de primitives de mouvement crée un mouvement de préhension avec au moins deux degrés de liberté.

14. Système de commande de préhension (100) selon la revendication 8, dans lequel la chaîne de primitives de mouvement sont l'une parmi des chaînes de système prédéfinies, des chaînes définies par l'utilisateur, et définies dynamiquement par l'utilisateur.

15. Système de commande de préhension (100) selon la revendication 8, dans lequel le capteur de mouvement musculaire (101) est un capteur de signal d'électromyographie, EMG.
